# EUROPEAN PATENT APPLICATION

(11) **EP 3 579 239 A1**
(43) Date of publication of application: **11.12.2019**
(21) Application number: 18747413.5
(22) Date of filing: 31.01.2018
(51) Int. Cl.: G16H 10/00, G06Q 30/06

(54) **WRITTEN INSTRUCTION ISSUING SYSTEM, WRITTEN INSTRUCTION ISSUING METHOD, AND PROGRAM**

(30) Priority: 02.02.2017 JP 2017017686; 02.02.2017 JP 2017017742
(71) Applicant: DSi Corporation, Chuo-ku Tokyo 104-0061 (JP)
(72) Inventor: SATO Hiroshi, Tokyo 104-0061 (JP)
(74) Representative: Granleese, Rhian Jane
(86) International application number: PCT/JP2018/003266
(87) International publication number: WO 2018/143292

(57) **Abstract**

To provide a labo slip ordering system, a labo slip ordering method, and a program by which labo slips for dental laboratories can be accurately and easily ordered and efficient and low-cost orders can be realized. The labo slip ordering system includes a dental clinic interface unit that includes a dental technical product list display part for selecting a dental technical product and an ordering part that orders a labo slip for producing the dental technical product which is selected from the dental technical product list display part. The dental clinic interface unit further includes an other-name list in which a standard name of the dental technical product is associated with another name of the dental technical product. The dental technical product list display part enables the dental technical product to be selected depending on either the standard name or the other name.

## Description

### [TECHNICAL FIELD]

The present invention relates to a labo slip ordering system, a labo slip ordering method, and a program and especially relates to a technique for accurately and easily ordering labo slips for dental laboratories and realizing efficient and low-cost orders.

### [BACKGROUND ART]

Order placement and order reception between a dental clinic and a dental laboratory have been conventionally performed by using labo slips. A labo slip is a document which is ordered from a dental clinic and on which a name of a patient, a name of a dental technical product and a specification (which can include instructions on a used material and a production method, for example) of the dental technical product, a name of a dental clinic which is an ordering source, a name of a dental laboratory which is an ordering destination, and the like are written. In general, a dental clinic makes a labo slip by handwriting required information on a ready-made labo slip paper.

Patent Literature 1 describes a computer system allowing for performing order reception/placement of prostheses (corresponding to the above-mentioned dental technical product) among a plurality of dental clinics and a plurality of dental laboratories.

### [PRIOR ART LITERATURE]

### [PATENT LITERATURE]

[Patent Literature 1] Japanese Patent Application Laid-Open No. 2016-071784

### [SUMMARY OF THE INVENTION]

### [PROBLEMS TO BE SOLVED BY THE INVENTION]

The system described in Patent Literature 1 improves convenience by computer-systemizing a conventional order reception/placement task executed in a written form. The system described in Patent Literature 1, however, does not solve various problems occurring in the conventional order reception/placement task.

For example, a dental technical product is sometimes called with different names depending on dental laboratories. Therefore, dental clinics have been required to make labo slips while separately using different names of a dental technical product for respective dental laboratories which are ordering destinations. Further, as production costs and delivery dates of a dental technical product are different among dental laboratories, it has been difficult to appropriately select an ordering destination while comparing the production costs and the delivery dates. Furthermore, labo slips are obliged to be made as paper documents. Therefore, even though the system of Patent Literature 1 is used, dental clinics cannot avoid conventionally making labo slips by handwriting, requiring much labor and time.

The present invention is made to solve problems as those mentioned above and an object of the present invention is to provide a labo slip ordering system, a labo slip ordering method, and a program by which labo slips for dental laboratories can be accurately and easily ordered and efficient and low-cost orders can be realized.

### [MEANS TO SOLVE THE PROBLEMS]

A labo slip ordering system according to an embodiment of the present invention is characterized in that the labo slip ordering system includes a dental clinic interface unit that includes a dental technical product list display part for selecting a dental technical product and an ordering part that prints a labo slip for producing the dental technical product which is selected from the dental technical product list display part, in which the ordering part prints an identifier for uniquely identifying the labo slip on the labo slip, and at least any one of receipt data, patient data, and chart data can be referred to by using the identifier.

A labo slip ordering method according to another embodiment is characterized in that the labo slip ordering method includes: a display step for displaying, by a computer, a dental technical product list for selecting a dental technical product; and an ordering step for printing, by the computer, a labo slip for producing the dental technical product which is selected from the dental technical product list, in which an identifier for uniquely identifying the labo slip is printed on the labo slip in the ordering step, and at least any one of receipt data, patient data, and chart data can be referred to by using the identifier.

A program according to still another embodiment is a program for making a computer execute the above-described labo slip ordering method.

A labo slip ordering system according to an embodiment of the present invention is characterized in that the labo slip ordering system includes a dental clinic interface unit that includes a dental technical product list display part for selecting a dental technical product and an ordering part that orders a labo slip for producing the dental technical product which is selected from the dental technical product list display part, in which the dental clinic interface unit further includes an other-name list in which a standard name of the dental technical product is associated with another name of the dental technical product, and the dental technical product list display part enables the dental technical product to be selected depending on either the standard name or the other name.

The labo slip ordering system according to another embodiment is characterized in that the dental clinic interface unit further includes a technical type selection part for selecting a major class of the dental technical product and an insurance/own-expense selection part for selecting insurance or own expense, and the dental technical product list display part enables selection of only the dental technical product which meets the major class and a result of the selection between insurance or own expense.

The labo slip ordering system according to still another embodiment is characterized in that the dental clinic interface unit further includes an ordering destination selection part that is capable of displaying a charge and a delivery date of the dental technical product for each of a plurality of dental laboratories which can be an ordering destination of the dental technical product.

The labo slip ordering system according to yet another embodiment is characterized in that the ordering part prints a paper document on which a content of the labo slip and an identifier, by which the labo slip can be uniquely identified, are written.

The labo slip ordering system according to yet another embodiment is characterized in that the labo slip ordering system further includes: a labo slip administration unit that stores the labo slip with status information; and a dental laboratory interface unit that includes a labo slip status change part which is capable of changing the status information.

An labo slip ordering method according to yet another embodiment is characterized in that the labo slip ordering method includes: a display step for displaying, by a computer, a dental technical product list for selecting a dental technical product; and an ordering step for ordering, by the computer, a labo slip for producing the dental technical product which is selected from the dental technical product list, in which the dental technical product can be selected depending on either a standard name or another name by referring to an other-name list in which the standard name of the dental technical product is associated with the other name of the dental technical product, in the display step.

A program according to yet another embodiment is a program for making a computer execute the above-described labo slip ordering method.

### [EFFECTS OF THE INVENTION]

According to the present invention, a labo slip ordering system, a labo slip ordering method, and a program can be provided by which labo slips for dental laboratories can be accurately and easily ordered and efficient and low-cost orders can be realized.

### [BRIEF DESCRIPTION OF THE DRAWINGS]

Fig. 1 is a block diagram illustrating a configuration of a labo slip ordering system 100.
Fig. 2 is a flowchart illustrating an operation of the labo slip ordering system 100.
Fig. 3 is a diagram illustrating a screen example of a dental clinic interface unit 110.
Fig. 4A is a diagram illustrating a screen example of the dental clinic interface unit 110.
Fig. 4B is a diagram illustrating a screen example of the dental clinic interface unit 110.
Fig. 4C is a diagram illustrating a screen example of the dental clinic interface unit 110.
Fig. 5 is a diagram illustrating a screen example of a dental laboratory interface unit 130.
Fig. 6 is a diagram illustrating a screen example of the dental laboratory interface unit 130.
Fig. 7 is a diagram illustrating a screen example of the dental clinic interface unit 110.
Fig. 8 is a diagram illustrating an example of status information.
Fig. 9 is a diagram illustrating an example of an other-name list.

### [DETAILED DESCRIPTION OF THE EMBODIMENT]

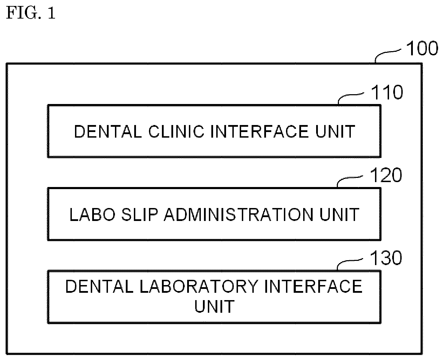

A specific embodiment to which the present invention is applied is described in detail below with reference to the accompanying drawings. An labo slip ordering system 100 according to the embodiment of the present invention is first described with reference to the block diagram in Fig. 1.

The labo slip ordering system 100 includes a dental clinic interface unit 110, a labo slip administration unit 120, and a dental laboratory interface unit 130. The labo slip ordering system 100 is an information processing device which typically executes a program, which is read from a storage device by a central processing unit (CPU), so as to logically realize processing units such as the dental clinic interface unit 110, the labo slip administration unit 120, and the dental laboratory interface unit 130. Various hardware configurations are conceivable to realize the labo slip ordering system 100. For example, a configuration may be employed in which all of these processing units are mounted on one or a plurality of servers and the server includes the labo slip administration unit 120 and provides functions of the dental clinic interface unit 110 and the dental laboratory interface unit 130 when the server is remotely accessed from a terminal device of each of a dental clinic and a dental laboratory. Alternatively, the functions of the dental clinic interface unit 110 and the dental laboratory interface unit 130 may be respectively mounted on terminal devices of a dental clinic and a dental laboratory and these terminal devices and a server may be communicably connected with each other, realizing the labo slip ordering system 100.

The dental clinic interface unit 110 provides a function for inputting items to be written in a labo slip, a function for registering labo slip data in the labo slip administration unit 120, a function for outputting labo slip documents, a function for acquiring and changing status information of labo slips, and so forth, for dental clinics.

The labo slip administration unit 120 stores contents of labo slips made by the dental clinic interface unit 110. Further, the labo slip administration unit 120 holds status information of labo slips and changes the status information in response to a request from the dental clinic interface unit 110 or the dental laboratory interface unit 130.

The labo slip administration unit 120 preferably issues a unique identifier (referred to as a labo slip ID) to labo slip data created by the dental clinic interface unit 110 and stores the labo slip ID and the labo slip data in a manner to associate the labo slip ID with the labo slip data. More preferably, a labo slip ID is associated with at least one of an identifier of receipt data (referred to as a receipt ID), an identifier of patient data (referred to as a patient ID), and an identifier of chart data (referred to as a chart ID). Receipt data is a bill data for medical fee. Receipt data is typically created by a receipt computer and stored in a predetermined storage region in a manner to be added with a receipt ID. If labo slip IDs and receipt IDs are associated with each other, labo slip data related to a receipt can be searched based on the receipt data and receipt data related to a labo slip can be searched based on the labo slip data, for example. Similar advantageous effects can be obtained for patient data and chart data by associating labo slip IDs with each of patient IDs and chart IDs. There are various conceivable methods for performing the above-mentioned association. For example, when the dental clinic interface unit 110 creates labo slip data, related receipt ID, patient ID, or chart ID may be included in the labo slip data. Alternatively, the labo slip administration unit 120 may create a table, for example, which represents a correspondence relation between a labo slip ID and a related receipt ID, patient ID, or chart ID. Also, a receipt computer or the like may create the table.

The dental laboratory interface unit 130 provides a function for acquiring contents of labo slips, a function for acquiring and changing status information of labo slips, and so forth, for dental laboratories.

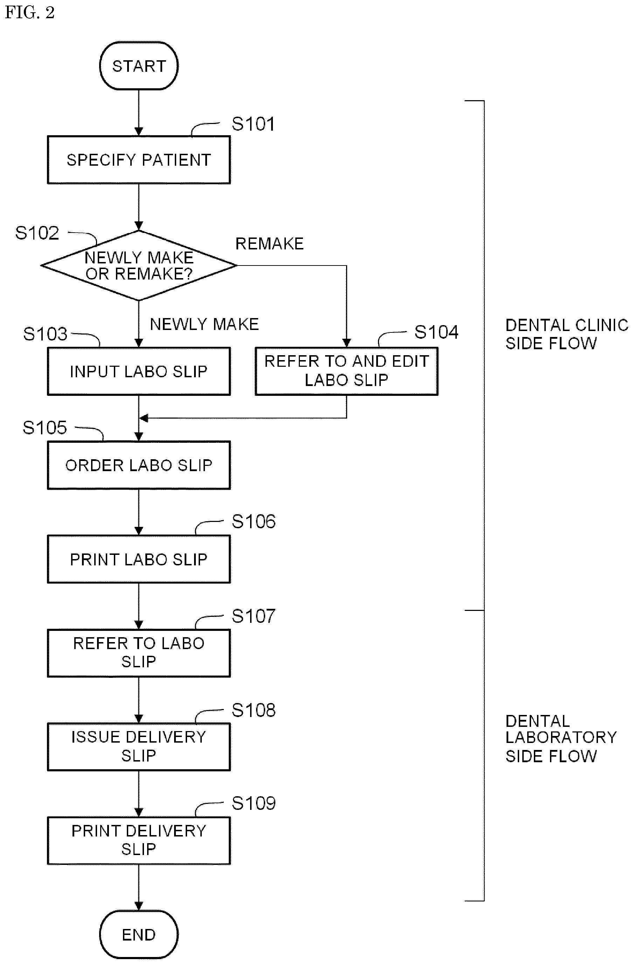

An example of an operation of the labo slip ordering system 100 is now described with reference to the flowchart in Fig. 2. Here, steps S101 to S106 mainly constitute a dental clinic side processing flow performed by the dental clinic interface unit 110 and steps S107 to S109 mainly constitute a dental laboratory side processing flow performed by the dental laboratory interface unit 130.

### <Dental clinic side processing flow>

### S101: Patient specification

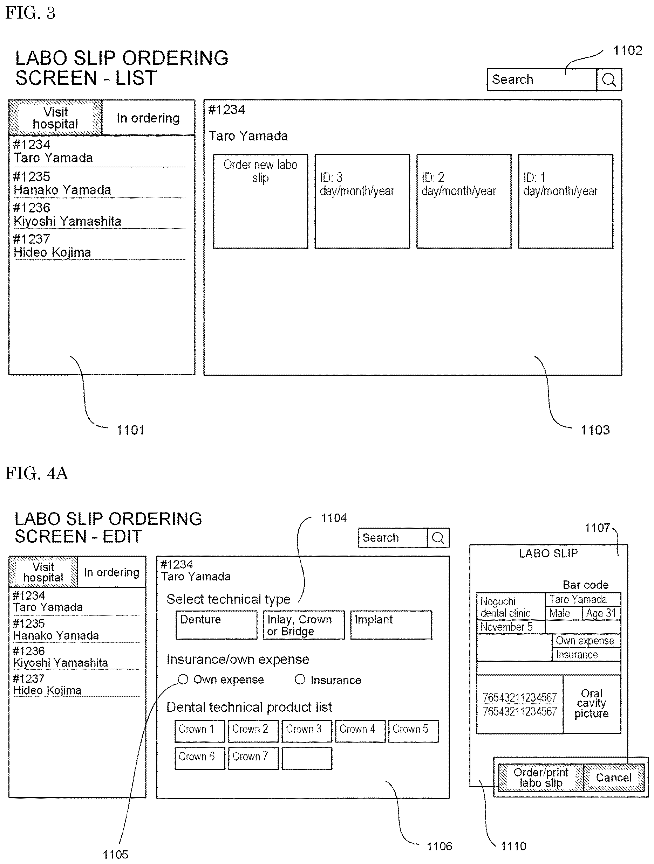

The dental clinic interface unit 110 allows a terminal device in a dental clinic to display a patient specifying screen. Fig. 3 illustrates an example of the patient specifying screen.

A patient list display part 1101 displays a list of patients meeting a predetermined condition. The dental clinic interface unit 110 refers to patient database which is administrated by a receipt computer, an electronic chart system, or the like, which is not illustrated, the labo slip administration unit 120, and so forth, extracts patients meeting a predetermined condition, and displays a list of the patients on the patient list display part 1101. The dental clinic interface unit 110 can use various information stored in the patient database, such as a patient name, a patient number, a sex, an age, a birthday, an address, a phone number, date and time of past hospital visit, and date and time of scheduled hospital visit, as extraction conditions. Further, an ordering date of a labo slip, a scheduled delivery date of a dental technical product related to the labo slip, a delivery date, and the like, which are stored in the labo slip administration unit 120 may be used as extraction conditions.

Extraction conditions may be specified by various methods. For example, the patient list display part 1101 in Fig. 3 includes a plurality of tabs: "visit hospital", "in ordering", and so forth. The dental clinic interface unit 110 extracts patients who are scheduled to visit a hospital on the day when the "visit hospital" tab is selected. When the "in ordering" tab is selected, patients whose labo slips have been already ordered but delivery dates have not been registered yet are extracted. Further, the dental clinic interface unit 110 may extract patients by using an arbitrary word inputted in a search box 1102 as a key. The dental clinic interface unit 110 can also extract patients based on arbitrary search conditions by using another arbitrary search interface. The dental clinic interface unit 110 displays a list of patients thus extracted on the patient list display part 1101.

On a labo slip list display part 1103, labo slips ordered in the past for a specific patient are displayed as a list. When a user selects one patient displayed on the patient list display part 1101, the dental clinic interface unit 110 refers to the labo slip administration unit 120, extracts past labo slip data of the selected patient, and displays information by which the extracted labo slip data can be identified, such as an ordering date, on the labo slip list display part 1103 as a list. The dental clinic interface unit 110 also displays a direction interface for starting making a new labo slip. This corresponds to an "order new labo slip" button in the example in Fig. 3.

### S102: Patient specification

When a past labo slip is selected on the labo slip list display part 1103, the processing goes to step S104. On the other hand, when "order new labo slip" is selected, the processing goes to step S103.

### S103: Labo slip input

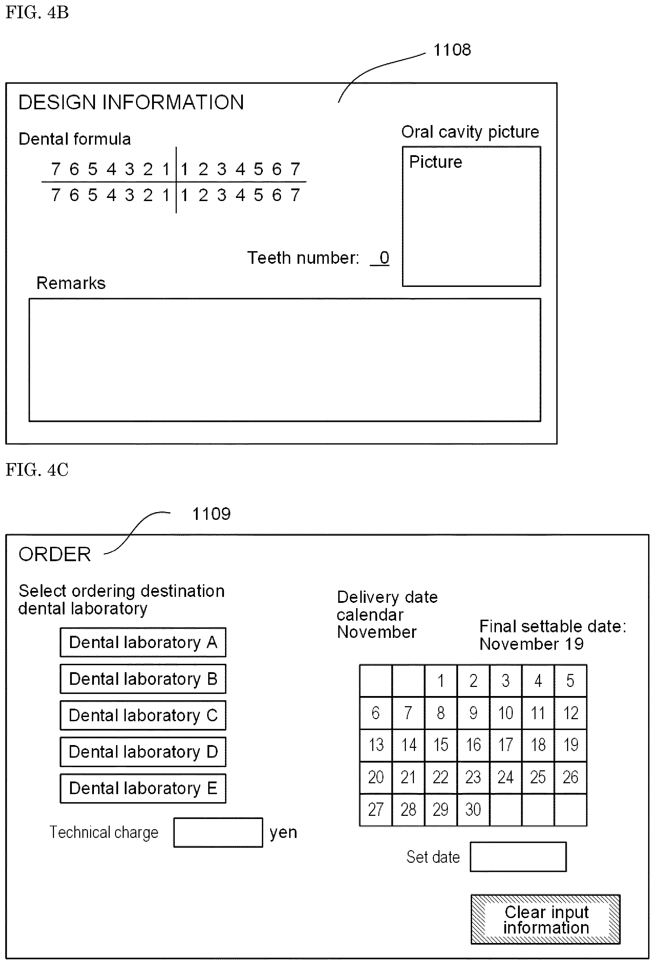

The dental clinic interface unit 110 receives an input of necessary information so as to newly make a labo slip related to a patient specified in step S101. Fig. 4A to Fig. 4C illustrate an example of a labo slip input screen.

A technical type selection part 1104 is an interface for specifying a major class of dental technical products. Buttons for selecting one from three types, "denture", "inlay, crown or bridge", and "implant", are arranged in this example.

An insurance/own-expense selection part 1105 is an interface for specifying presence/absence of insurance coverage. Radio buttons for selecting one between "insurance" and "own expense" are aligned in this example.

A dental technical product list display part 1106 is an interface for specifying a minor class of dental technical products. The dental clinic interface unit 110 displays a list of dental technical products, which belong to the major class selected in the technical type selection part 1104 and correspond to presence/absence of insurance coverage selected in the insurance/own-expense selection part 1105, on the dental technical product list display part 1106. For example, dental technical products such as "crown", "composite resin", "porcelain fused to metal crown(PFM)", and "all ceramic crown" as minor classes belong to a major class "inlay, crown or bridge". Here, "crown" and "composite resin" can be covered by insurance, but "porcelain fused to metal crown(PFM)" and "all ceramic crown" are not covered by insurance. If "inlay, crown or bridge" is selected as a major class and "insurance" is selected in distinction between insurance and own expense, the dental clinic interface unit 110 extracts only minor classes "crown" and "composite resin", which meet all these conditions, and displays a list thereof on the dental technical product list display part 1106. Buttons for selecting one from a plurality of minor classes are arranged in the example in Fig. 4A.

Here, the dental clinic interface unit 110 may include another arbitrary means for selecting a dental technical product together with or instead of the above-described technical type selection part 1104, insurance/own-expense selection part 1105, and dental technical product list display part 1106. For example, the dental clinic interface unit 110 may include a means for searching a dental technical product, a means for permitting selection of a desired dental technical product from a list of dental technical products frequently ordered, and so forth. Alternatively, a narrowing search means based on other arbitrary conditions in addition to or instead of technical types and whether to be insurance or own expense may be provided.

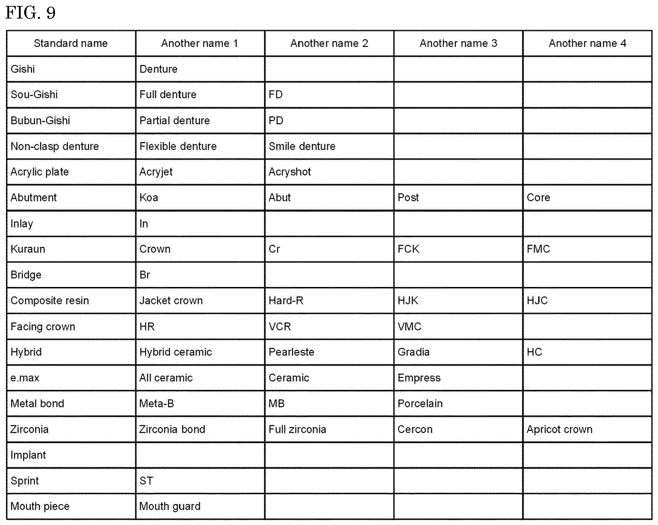

Here, different names (other names) are sometimes used even for identical dental technical products depending on dental laboratories and dental clinics. For example, there is a case where a standard dental technical product name "crown" is called "Cr" by dental laboratory A and is called "FMC" by dental laboratory B. Therefore, dental clinics have conventionally separately used different names for a dental technical product written on labo slips depending on ordering destinations. Fig. 9 illustrates examples of standard names and other names of dental technical products.

In the present embodiment, the dental clinic interface unit 110 can preliminarily hold a list of other names as the one illustrated in Fig. 9. For example, "Cr" and "FMC" are held in an other-name list corresponding to a standard name "crown". When a minor class "crown" is selected, the dental clinic interface unit 110 can display the other-name list and allow a user to select one name among other names. Here, it is preferable to adopt standard names based on names of dental technical products described in the insurance points table issued by the Ministry of Health, Labor and Welfare. Further, the dental clinic interface unit 110 or the dental laboratory interface unit 130 may include an interface for arbitrarily registering other names corresponding to standard names.

That is, the dental clinic interface unit 110 needs to preliminarily hold a major class to which a dental technical product belongs, whether or not insurance is applied, and preferably an other-name list in a manner to associate these with each dental technical product of a minor class. In the present embodiment, it is assumed that the dental clinic interface unit 110 preliminarily stores these pieces of information in a storage region which is not illustrated.

A labo slip format display part 1107 displays contents of a labo slip which are presumed based on information which have been inputted up to the present time point. The dental clinic interface unit 110 acquires a name, age, sex, and the like of a patient from patient database, for example, and displays the name, age, sex, and the like of the patient on a predetermined position in the labo slip format display part 1107. The dental clinic interface unit 110 further displays whether to be insurance or own expense selected in the insurance/own-expense selection part 1105 and a name of a dental technical product selected in the dental technical product list display part 1106 on a predetermined position in the labo slip format display part 1107.

Here, the labo slip ordering system 100 may preliminarily hold dental clinic information in a storage region which is not illustrated. In this case, the dental clinic interface unit 110 may acquire arbitrary information included in the dental clinic information and display the information on a predetermined position in the labo slip format display part 1107. The dental clinic information can include a name, address, phone number, and the like of a dental clinic, for example. The dental clinic interface unit 110 may separately include an interface for registering dental clinic information.

A design information input part 1108 is an interface for inputting design information of a dental technical product. For example, the dental clinic interface unit 110 reflects numbers, which are selected by a user in a dental formula input column, on the labo slip format display part 1107. Further, the number of selected teeth is counted and the counted number is reflected on a teeth number column.

The dental clinic interface unit 110 may accept an input of an oral cavity picture. For example, when a user selects an oral cavity picture column, the dental clinic interface unit 110 activates a camera function of a dental clinic terminal. The dental clinic interface unit 110 acquires and stores a picture taken by the camera and displays a thumbnail of the picture on the oral cavity picture column. Alternatively, the dental clinic interface unit 110 may provide an interface for selecting an image which is preliminarily stored in a dental clinic terminal or another storage region, acquire and store the selected image, and display the thumbnail of the image on the oral cavity picture column.

Further, the dental clinic interface unit 110 may include a remarks column for inputting detailed direction contents. For example, when a dental clinic desires to specify a material of a dental technical product, the dental clinic can write the specified contents in this remarks column.

An ordering destination selection part 1109 is an interface for selecting an ordering destination while comparing charges, delivery dates, and the like. The ordering destination selection part 1109 displays a plurality of dental laboratories which are candidates of an ordering destination, in the example in Fig. 4C. A charge display column and a delivery date calendar are also provided. The ordering destination selection part 1109 further holds a technical charge for every dental technical product and the earliest delivery date of every dental technical product for every dental laboratory, in a storage region which is not illustrated. When a user selects one dental laboratory, the ordering destination selection part 1109 acquires a technical charge and a delivery date of the selected dental laboratory from the storage region and displays the technical charge and the delivery date on the charge display column and the delivery date calendar respectively. The technical charge and the delivery date correspond to a dental technical product selected in the dental technical product list display part 1106 and design information inputted in the design information input part 1108. For example, in ordering two dental technical products "crowns", the ordering destination selection part 1109 can calculate a charge for two crowns based on a unit value of the "crown" which is preliminarily stored and display the charge on the charge display column. Further, a configuration may be employed by which a dental clinic can arbitrarily change a delivery date displayed on the delivery date calendar.

### S104: Labo slip editing

When a past labo slip is selected in the labo slip list display part 1103, the dental clinic interface unit 110 refers to the labo slip administration unit 120 and reads data of the selected labo slip. Then, screens similar to those in Fig. 4A to Fig. 4C are displayed in a state that contents of the selected labo slip are described. The dental clinic interface unit 110 accepts change, performed by a user, in the described contents of the labo slip.

### S105: Labo slip ordering

An ordering part 1110 includes an ordering button and a labo slip printing button. These two buttons may be integrated to one. When the ordering button is pressed, the ordering part 1110 stores information, which is inputted through the above-described series of processing, in a predetermined storage region of the labo slip administration unit 120 to order a labo slip. Information administrated by the labo slip administration unit 120 may include not only information for specifying a dental technical product and a specification of the dental technical product but also an ordering date, a scheduled delivery date, whether to be insurance or own expense, patient information, information of a dental clinic which is an ordering source, information of a dental laboratory which is an ordering destination, and the like.

Here, the labo slip ordering system 100 may preliminarily hold dental laboratory information in a storage region which is not illustrated. Dental laboratory information may include names, addresses, phone numbers, and the like of dental laboratories, for example. In this case, the dental clinic interface unit 110 may acquire arbitrary information included in the dental laboratory information and store the acquired information in the labo slip administration unit 120 as a part of information constituting a labo slip. The dental laboratory interface unit 130 may include an interface for registering dental laboratory information.

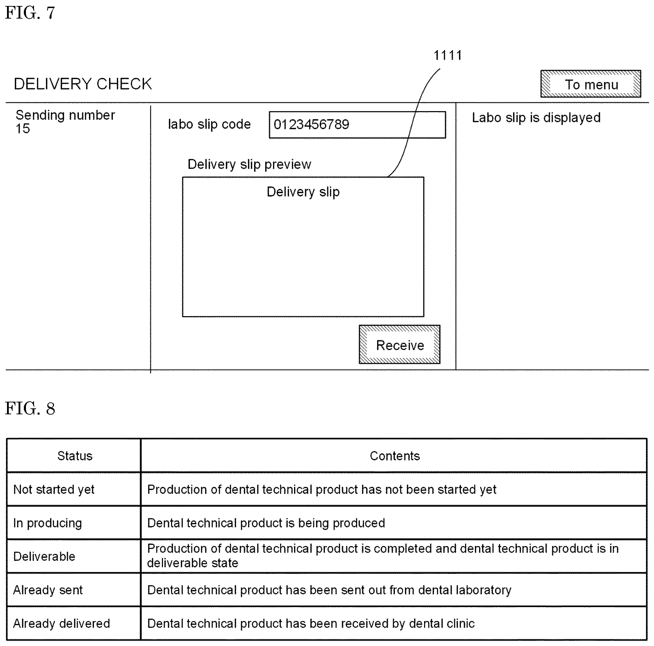

When a labo slip is ordered by the dental clinic interface unit 110, the labo slip administration unit 120 generates an identifier for uniquely identifying the labo slip, that is, a labo slip ID, and stores the labo slip and the labo slip ID in an associated manner. Further, the labo slip administration unit 120 stores status information while associating the status information with every labo slip. Fig. 8 illustrates an example of status information. According to this example, a status of a labo slip ordered by the dental clinic interface unit 110 is "not started yet".

### S106: Labo slip printing

When the labo slip printing button is pressed, the ordering part 1110 prints a labo slip. The ordering part 1110 preferably performs printing in a similar format to that of the labo slip format display part 1107. Information to be printed on a labo slip is to be based on the same information as the one stored in the labo slip administration unit 120. Further, the ordering part 1110 preferably prints a labo slip ID, another identifier corresponding to the labo slip ID, a bar code in which the labo slip ID or the above-mentioned identifier is encoded, or the like, on a labo slip. A printed labo slip is generally sent to a dental laboratory with a model. Further, the ordering part 1110 may notify a dental laboratory of the above-mentioned labo slip ID and the above-mentioned identifier by another arbitrary means.

Here, the ordering part 1110 may include a cancel button. When the cancel button is pressed, the dental clinic interface unit 110 discards information which have been inputted up to the current time and the processing goes to step S101.

### <Dental laboratory side processing flow>

### S107: Labo slip reference

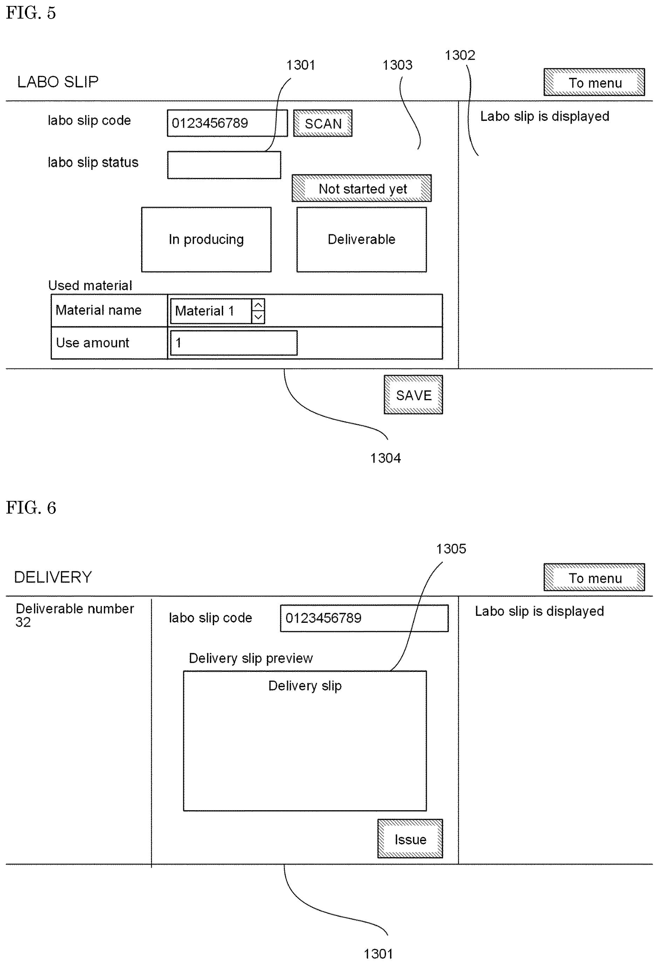

The dental laboratory interface unit 130 accepts selection of labo slip data in a terminal device in a dental laboratory. A labo slip display screen illustrated in Fig. 5 includes an input box 1301 for inputting a labo slip ID or an identifier corresponding to the labo slip ID. The dental laboratory interface unit 130 extracts corresponding labo slip data from the labo slip administration unit 120 by using the above-mentioned labo slip ID or the above-mentioned identifier, which is inputted in the input box 1301, as a key and displays contents of the labo slip data on a labo slip display column 1302. Here, labo slip data may be selected in a manner such that a bar code scanner, which is not illustrated, reads a bar code drawn on a printed labo slip to output the above-mentioned labo slip ID or the above-mentioned identifier and the dental laboratory interface unit 130 extracts a labo slip from the labo slip administration unit 120 based on the labo slip ID or the identifier, for example. Alternatively, a configuration may be employed in which labo slips extracted from the labo slip administration unit 120 based on a predetermined condition are displayed as a list so as to allow a user to select a desired labo slip.

The dental laboratory interface unit 130 accepts change in status information of a labo slip on a labo slip status change part 1303. In the present embodiment, the labo slip status change part 1303 includes two buttons: "in producing" and "deliverable". For example, when a user in a dental laboratory starts producing a dental technical product based on a labo slip, the user presses the "in producing" button. In response, the dental laboratory interface unit 130 rewrites status information of this labo slip stored in the labo slip administration unit 120 to "in producing". When a user completes producing the dental technical product and presses the "deliverable" button, the dental laboratory interface unit 130 rewrites the status information of this labo slip stored in the labo slip administration unit 120 to "deliverable".

The labo slip display screen may include a used material entry part 1304. The used material entry part 1304 accepts an input of information on a name of a used material, a use amount, and the like. The dental laboratory interface unit 130 allows the labo slip administration unit 120 to store the inputted information so that the information is associated with a labo slip. Status information and information related to a used material are preferably stored in the labo slip administration unit 120 in response to press of the "save" button.

### S108: Delivery slip issuing

Fig. 6 illustrates an example of a delivery screen provided by the dental laboratory interface unit 130. The dental laboratory interface unit 130 reads out labo slip data, whose status information is "deliverable" and which is specified by a user, for example, among labo slip data stored in the labo slip administration unit 120 so as to make a delivery slip 1305. The dental laboratory interface unit 130 may extract a labo slip whose status information is "deliverable" and which has a labo slip ID or another identifier corresponding to the labo slip ID, which is inputted in the input box, or extract a labo slip whose status information is "deliverable" and which has the above-mentioned labo slip ID or the above-mentioned identifier, which is outputted by a bar code scanner which is not illustrated, for example. Alternatively, the dental laboratory interface unit 130 may be configured so that the dental laboratory interface unit 130 displays a list of labo slips whose status information is "deliverable" on a delivery screen so as to allow a user to select a desired labo slip. Items to be described on a delivery slip are typically the same as items described in a labo slip. A delivery date and information related to a used material inputted in the used material entry part 1304 may be additionally described.

The dental laboratory interface unit 130 stores delivery slip data in the labo slip administration unit 120 in response to press of the "issue" button, for example. At this time, the dental laboratory interface unit 130 stores the above-mentioned labo slip data specified by a user and this delivery slip data in an associated manner to issue a delivery slip. When a delivery slip is issued by the dental laboratory interface unit 130, the labo slip administration unit 120 generates an identifier (delivery slip ID) for uniquely identifying a delivery slip and stores the identifier and the delivery slip in an associated manner.

### S109: Delivery slip printing

At this time, the dental laboratory interface unit 130 preferably prints a delivery slip. Information to be printed on a delivery slip is to be based on the same information as the one in a delivery slip stored in the labo slip administration unit 120. Further, the dental laboratory interface unit 130 preferably prints a delivery slip ID, another identifier corresponding to the delivery slip ID, a bar code in which the delivery slip ID or the above-mentioned identifier is encoded, or the like, on a delivery slip. A printed delivery slip is generally sent to a dental clinic with a dental technical product. Further, the dental laboratory interface unit 130 may notify a dental clinic of the above-mentioned identifier by another arbitrary means. At this time, the dental laboratory interface unit 130 changes status information of a labo slip related to the delivery slip to "already sent", for example.

Fig. 7 illustrates an example of a delivery check screen which is used on a dental clinic side receiving a dental technical product. The dental clinic interface unit 110 provides the delivery check screen. The dental clinic interface unit 110 reads out a delivery slip, which is associated with a labo slip whose status information is "already sent", for example, among labo slips stored in the labo slip administration unit 120 and which is specified by a user, and displays the delivery slip on a delivery slip display part 1111. For example, the dental laboratory interface unit 130 may extract a delivery slip that is associated with a labo slip whose status information is "already sent" and has the above-mentioned delivery slip ID or the above-mentioned identifier, which is inputted into the input box, or extract a delivery slip that is associated with a labo slip whose status information is "already sent" and has the delivery slip ID or the above-mentioned identifier, which is outputted by a bar code scanner which is not illustrated. Alternatively, a configuration may be employed in which the dental clinic interface unit 110 displays a list of delivery slips which are associated with labo slips whose status information is "deliverable" on the delivery check screen so as to allow a user to select a desired delivery slip.

Then, the dental clinic interface unit 110 changes the status of the labo slip associated with the above-mentioned selected delivery slip into "already delivered" in response to press of a "receive" button performed by a user, for example.

According to the present embodiment, the labo slip ordering system 100 preliminarily grasps other names of dental technical products which are called in different ways depending on a dental laboratory and thus holds a correspondence relation between a standard name and other names of a dental technical product. Accordingly, it is not necessary to make labo slips while separately using different dental technical product names for respective dental laboratories which are ordering destinations, and labo slips can be accurately, easily, and efficiently made.

Further, though a production cost and a delivery date of a dental technical product vary depending on a dental laboratory, the labo slip ordering system 100 holds charges and delivery dates for respective dental laboratories and can easily compare charges and delivery dates for producing a dental technical product described in an inputted labo slip. Thus, a dental clinic can easily order a dental technical product at a low cost.

Further, in the present embodiment, labo slips inputted into the labo slip ordering system 100 can be outputted as paper documents. Accordingly, labo slips do not have to be made by handwriting as the conventional way, being able to reduce much labor and time.

Note that the present invention is not limited to the above-described embodiment and alterations may be appropriately made within the scope of the idea of the invention. For example, an identifier is also issued for a delivery slip in the above-described embodiment. However, the present invention is not limited to this, and there is no problem to employ a configuration in which a delivery slip is identified based on an identifier of a labo slip associated with the delivery slip.

Further, each processing means constituting the present invention may be configured by hardware and arbitrary processing may be logically realized by allowing a central processing unit (CPU) to execute a computer program. In this case, the computer program can be stored with various types of non-transitory computer readable media and supplied to a computer. Further, the program may be supplied to a computer with various types of transitory computer readable media. The transitory computer readable media include electrical signals, optical signals, and electromagnetic waves. The transitory computer readable media are capable of supplying the program to a computer via a wired communication path such as an electrical wire and an optical fiber or a wireless communication path.

### [DESCRIPTION OF REFERENCE NUMERALS]

- 100: labo slip ordering system
- 110: dental clinic interface unit
- 1101: patient list display part
- 1102: search box
- 1103: labo slip list display part
- 1104: technical type selection part
- 1105: insurance/own-expense selection part
- 1106: dental technical product list display part
- 1107: labo slip format display part
- 1108: design information input part
- 1109: ordering destination selection part
- 1110: ordering part
- 1111: delivery slip display part
- 120: labo slip administration unit
- 130: dental laboratory interface unit
- 1301: input box
- 1302: labo slip display column
- 1303: labo slip status change part
- 1304: used material entry part
- 1305: delivery slip

## Claims

1. A labo slip ordering system comprising:
a dental clinic interface unit that includes a dental technical product list display part for selecting a dental technical product and an ordering part that prints a labo slip for producing the dental technical product, the dental technical product being selected from the dental technical product list display part, wherein
the ordering part prints an identifier for uniquely identifying the labo slip on the labo slip, and
at least any one of receipt data, patient data, and chart data can be referred to by using the identifier.

2. A labo slip ordering method comprising:
a display step for displaying, by a computer, a dental technical product list for selecting a dental technical product; and
an ordering step for printing, by the computer, a labo slip for producing the dental technical product, the dental technical product being selected from the dental technical product list, wherein
an identifier for uniquely identifying the labo slip is printed on the labo slip in the ordering step, and
at least any one of receipt data, patient data, and chart data can be referred to by using the identifier.

3. A program for making a computer execute the labo slip ordering method according to Claim 2.

4. A labo slip ordering system comprising:
a dental clinic interface unit that includes a dental technical product list display part for selecting a dental technical product and an ordering part that orders a labo slip for producing the dental technical product, the dental technical product being selected from the dental technical product list display part, wherein
the dental clinic interface unit further includes an other-name list in which a standard name of the dental technical product is associated with another name of the dental technical product, and
the dental technical product list display part enables the dental technical product to be selected depending on either the standard name or the other name.

5. The labo slip ordering system according to Claim 4, wherein
the dental clinic interface unit further includes
a technical type selection part for selecting a major class of the dental technical product, and
an insurance/own-expense selection part for selecting insurance or own expense, and
the dental technical product list display part enables selection of only the dental technical product which meets the major class and a result of the selection between insurance or own expense.

6. The labo slip ordering system according to Claim 4, wherein
the dental clinic interface unit further includes an ordering destination selection part that is capable of displaying a charge and a delivery date of the dental technical product for each of a plurality of dental laboratories which can be an ordering destination of the dental technical product.

7. The labo slip ordering system according to Claim 4, wherein
the ordering part prints a paper document on which a content of the labo slip and an identifier, by which the labo slip can be uniquely identified, are written.

8. The labo slip ordering system according to Claim 4, further comprising:
a labo slip administration unit that stores the labo slip with status information; and
a dental laboratory interface unit that includes a labo slip status change part, the labo slip status change part being capable of changing the status information.

9. A labo slip ordering method comprising:
a display step for displaying, by a computer, a dental technical product list for selecting a dental technical product; and
an ordering step for ordering, by the computer, a labo slip for producing the dental technical product, the dental technical product being selected from the dental technical product list, wherein
the dental technical product can be selected depending on either a standard name or another name by referring to an other-name list in which the standard name of the dental technical product is associated with the other name of the dental technical product, in the display step.

10. A program for making a computer execute the labo slip ordering method according to Claim 9.
